# EUROPEAN PATENT APPLICATION

(11) **EP 1 927 349 A1**
(43) Date of publication of application: **04.06.2008**
(21) Application number: 06798179.5
(22) Date of filing: 21.09.2006
(51) Int. Cl.: A61K 31/05, A61K 31/122, A61P 43/00

(54) **COMPOSITION FOR LIFE EXTENSION AND METHOD OF EXTENDING THE LIFE**

(30) Priority: 22.09.2005 JP 2005276816
(71) Applicant: KANEKA CORPORATION, Osaka-shi, Osaka 530-8288 (JP)
(72) Inventor: KISHIDA, Hideyuki, Kakogawa-shi, Hyogo 675-0039 (JP); KAWABE, Taizou, Himeji-shi, Hyogo 672-8044 (JP); HOSOE, Kazunori, Takasago-shi, Hyogo 676-0025 (JP)
(74) Representative: Gillet, Raphaëlle
(86) International application number: PCT/JP2006/318687
(87) International publication number: WO 2007/034852

(57) **Abstract**

The present invention relates to a composition for life extension, comprising reduced coenzyme Q₁₀ as an active ingredient, as well as relates to a method of extending a life span using the composition comprising reduced coenzyme Q₁₀ as an active ingredient. In the present invention, it has been found that long-term ingestion of feed that contains reduced coenzyme Q₁₀ enables the life span of senescence-accelerated model mice to be extended. No sign indicating toxicity is observed in the senescence-accelerated model mice that have been fed with reduced coenzyme Q₁₀ over a long period. Based on these findings, it was found that the composition containing reduce coenzyme Q₁₀ as an active ingredient has potential to be a composition for life extension with high safety, capable of being taken for a long period. The present invention provides a composition, which can extend the life span of humans or animals, enables them to spend active and healthy senectitudes and shows high safety during the ingestion over a long period.

## Description

### TECHNICAL FIELD

The present invention relates to a composition for life extension and a method of extending a life span using the composition.

### BACKGROUND ART

Extension of a life span is one of significant issues for human beings. Recently, the life span of human beings has been dramatically extended due to medical developments, improvements in environment or health, and the like. However, extension of a life span is coming to be saturated in these days. The same goes for mammals other than humans, such as pets. Advanced age mammals including humans become infirm in the last several years of their life span in many cases. Even if the life is prolonged by medical treatments, it is not true happiness for mammals including humans to live a prolonged life under the condition with poor quality of life (QOL). In addition to these issues, how to extend not only the life span itself but also healthy life expectancy (namely, the number of years people can expect to live vigorously and actively in good health in our total life span) is one of significant issues in the present aging society.

In a latest study using animals, it has turned out that a life span is extensible with restricting ingesting energy (restricting calorie) (See the below Non-patent Document 1). However, long-period restriction of meals leads to lowering the quality of life for humans or pets, and it is not practical.

Based on the above study, a foodstuff composition, which showed a similar effect to the effect caused by caloric restriction, has been reported (See the below Patent Document 1). According to the report, L-carnitine, creatine, fatty acids (mono-unsaturated or polysaturated ones, especially ω-3 fatty acids), cardiolipin, nicotinamide or carbohydrates, and foodstuff compositions containing natural supply sources thereof prevent or restore functional disorders accompanied with aging of mammals without caloric restriction. However, any effect for extension of the life span of mammals has not been established indeed.

It is reported how to extend a life span by specifically controlling EF-2 kinase (See the below Patent Document 2). However, extension of a life span was evaluated only using nematodes, and actual effects for mammals, such as humans and pets have not been established yet. There is also a report about a method of extending a life span using special fats and oils (See the below Patent Document 3).

Effects in prevention or treatment of organ functional disorders, failing organs, and obesity, as well as sequelae thereof by administration of a compound which increases the level of ubiquinone (oxidized coenzyme Q) in the living body (See the below Patent documents 4) have been reported. It is also reported that administration of ubiquinone to rats that had been bred with unsaturated fatty acid-enriched feed extended the life span of the rat (See the below Non-patent Document 2). On the contrary, it is also reported that administration of ubiquinone did not affect the life span of mice in an experiment using mice (See the below Non-patent Document 1). It is also reported that ubiquinone and ubiquinol (reduced coenzyme Q) suppressed aging (See the below Patent Document 5).
Patent Document 1: JP 2004-519241 T
Patent Document 2: JP 2003-508347 T
Patent document 3: JP 2005-210978 A
Patent document 4: JP 2003-81873 T
Patent document 5: WO 2005/065672
Non-patent document 1: Free Radical Biology & Medicine, Vol.36, No 8, pp.1043-1057 (2004)
Non-patent literature 2: Experimental Gerontology, Vol.36, pp.189-194 (2004)

### DETAILED DESCRIPTION OF THE INVENTION

An object of the present invention is to provide a composition which can extend a life span without causing any side reaction.

As mentioned above, a certain report (See the above Non-patent Document 2) showed that oxidized coenzyme Q extended the life span of mammals, whereas another report showed that oxidized coenzyme Q did not extend the life span (See the above Non-patent Document 1). Regarding reduced coenzyme Q, prevention of aging by reduced coenzyme Q has already been reported. However, no teachings are found about extension of a life span by reduced coenzyme Q, and any other reports that mention such an effect cannot be found so far. In general, what is called an "anti-aging effect" (relaxation, improvement and inhibition of various symptoms accompanied with aging) and life extension, which substantially extends life span, is different from each other in their effects, and what has an anti-aging activity does not necessarily have a life extending effect. The present inventors have made intensive investigation, and as a result, found a definite life-extending effect in reduced coenzyme Q₁₀ (ubiquinol) for the first time. Based on this finding, they have completed the present invention.

That is, an aspect of the present invention is a composition for life extension, which comprises reduced coenzyme Q represented by the following general formula (1): 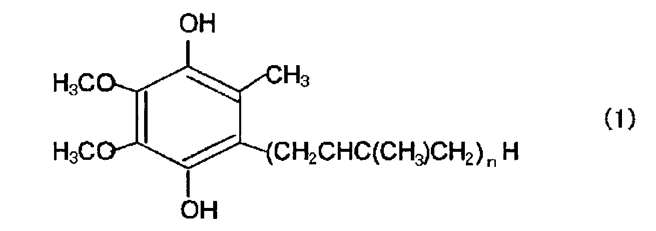 wherein n is an integer of 1 to 12; as an active ingredient.

Another aspect of the present invention is a method for extending a life span, which comprises
administering a composition comprising reduced coenzyme Q represented by the following general formula (1): 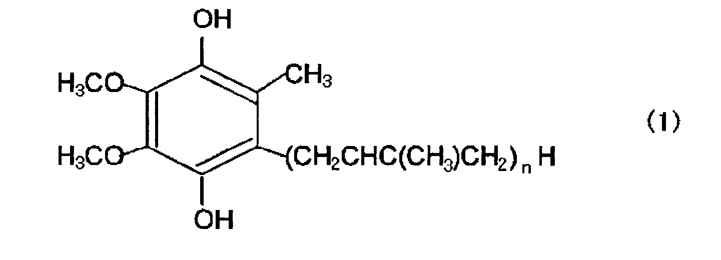 wherein n is an integer of 1 to 12; as an active ingredient.

If this invention is held from another side, the present invention is also referred to as a life-extending agent (life span-extending agent), which comprises the reduced coenzyme Q₁₀ represented by the above formula (1), as an active ingredient. In this case, a "composition for life extension" used herein may be read as a "life extending agent (life span-extending agent)".

Further, if this invention is held from another side, the present invention is also referred to as use of reduced coenzyme Q₁₀ represented by the above formula (1) for manufacturing a medicament for life extension (a medicament for extending a life span).

The composition for life extension of the present invention has an excellent effect of extending the life spans of animals, especially mammals. The composition for extending a life span of the present invention does not cause any side effect, and thus is highly safe.

### BEST MODE FOR CARRYING OUT THE INVENTION

Coenzyme Q is an essential component widely distributed in living organisms, from bacteria to mammals, and is known to occur as components of the electron transport system of mitochondria within the cells of living bodies. Coenzyme Q functions as an electron carrier in the electron transport system through repeating the cycle of oxidation-reduction in mitochondria. In addition, it is known that reduced coenzyme Q shows antioxidant activity. In humans, coenzyme Q₁₀, in which the side chain of the coenzyme Q has 10 repeating units, is the major component. As mentioned above, reduced coenzyme Q₁₀ shows antioxidant activity in vitro but oxidized coenzyme Q₁₀ does not show antioxidant activity. However, it is believed that oxidized coenzyme Q₁₀ is converted to the reduced form by means of reductase in vivo.

As an important feature of coenzyme Q₁₀, its high safety may be mentioned. It is reported that, in a chronic toxicity test in rats, no toxic effect was observed even when coenzyme Q₁₀ was administered every day over 52 weeks at 1,200 mg/kg/day (K. D. Williams et al., J. Agric. Food Chem. 47, 3756-3763, 1999). The dose of 1, 200 mg/kg/day, when converted to the human basis (body weight 50 kg), corresponds to 60 g/day. Since the usual dose of coenzyme Q₁₀ used as a health food in Europe and U.S.A. is 100 to 300 mg/day, it is evident that coenzyme Q₁₀ is a very highly safe supplement material.

Coenzyme Q has two different forms. One is reduced coenzyme Q (ubiquinol) represented by the following general formula (1): 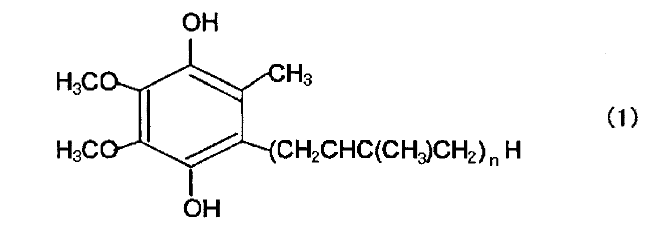 wherein n is an integer of 1 to 12. The other is oxidized coenzyme Q (ubiquinone) represented by the following general formula (2): 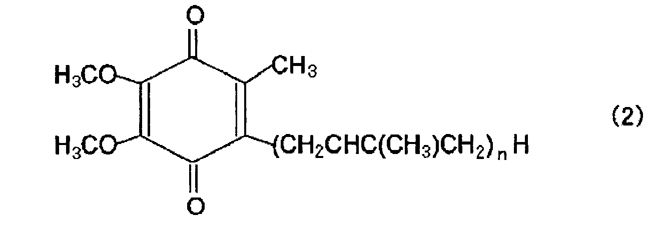 wherein n is an integer of 1 to 12. It has been known that about 40 to 90% of coenzyme Q usually exists as a reduced form in a living body. However, coenzyme Q conventionally used as common medicines and foodstuffs is oxidized coenzyme Q, because reduced coenzyme Q is easily oxidized by atmospheric oxygen. Oxidized coenzyme Q can be easily available as commercial products, or products obtainable by conventional processes, such as synthesis, fermentation, and extraction from natural sources.

The methods of obtaining reduced coenzyme Q are not particularly limited. Examples of the methods include a method comprising obtaining coenzyme Q containing oxidized coenzyme Q as the major ingredient in conventional processes such as synthesis, fermentation or extraction from natural sources, and then concentrating a reduced coenzyme Q fraction in the effluent by chromatography. In this case, an ordinary reducing agent such as sodium borohydride, sodium dithionite (sodium hydrosulfite), or ascorbic acid may optionally be added to the above coenzyme Q, and oxidized coenzyme Q in the coenzyme Q is reduced in a conventional manner to give reduced coenzyme Q, followed by chromatographic concentration. Reduced coenzyme Q can also be obtained by a method comprising reacting an existing highly pure oxidized coenzyme Q product with such a reducing agent as mentioned above. Alternatively, it is possible to utilize fungus bodies that contain reduced coenzyme Q.

Examples of ways to determine the ratio between oxidized forms and reduced forms in coenzyme Q include a method, which comprises assaying oxidized coenzyme Q and reduced coenzyme Q in a sample with a HPLC system provided with a UV detector, and then calculating the quantitative ratio between oxidized and reduced forms; and a method, which comprises calculating the ratio from peak areas each corresponding to oxidized coenzyme Q and reduced coenzyme Q assayed with a HPLC system provided with an electrochemical detector. The system provided with an electrochemical detector is highly useful in measuring the ratio of minute amount of the reduction form contained in a living body or a sample, since the electrochemical detector can specifically detects redox substances with high sensitivity. Specifically, the ratio may be measured with a HPLC analyzer (a product of Shimadzu), into which an electrochemical detector (Shiseido Co., Ltd) is built, under the following HPLC condition. An example of the condition is as follows: Column YMC-Pack (ODS-A303), Detection wavelength: 275 nm, Mobile phase: methanol (88%)/hexane (12%), Flow rate: 1 ml/min.

The composition for life extension of the present invention is a composition, which comprises a reduced coenzyme Q as an active ingredient. The composition may further comprise oxidized coenzyme Q as another active ingredient. That is, the composition may be a composition comprising coenzyme Q, which is a mixture of oxidized coenzyme Q and reduced coenzyme Q, as an active ingredient. In the cases where the coenzyme Q is a mixture of the reduced and oxidized forms, the amount of a reduced coenzyme Q is preferably not lower than 20% by weight, more preferably not lower than 40% by weight, still more preferably not lower than 50% by weight, relative to the total amount of coenzyme Q. Generally, the upper limit is preferably not higher than 99.5% by weight, but may be not higher than 95% by weight. If simply the term "coenzyme Q" is used herein, it means both reduced coenzyme Q alone and a mixture containing oxidized coenzyme Q and reduced coenzyme Q.

The reduced coenzyme Q or oxidized coenzyme Q which may be used in the present invention is a compound represented by the above general formula (1) or (2), wherein the number of the repeating units in the side chain (represented by n in the formulas) are 1 to 12. From a viewpoint that sufficient effects would be exerted in humans or many pets such as dogs, coenzyme Q having 10 repeating units in the side chain, i.e., reduced coenzyme Q₁₀ or oxidized coenzyme Q₁₀ can be suitably used.

The composition for life extension of the invention may contain, in addition to coenzyme Q, various additives acceptable from the medical or food hygiene law or the like viewpoint. In the cases where the composition is also used to treat various diseases, in addition to the life extension purpose, medicaments for treating the diseases may be used in combination.

The above-mentioned additives are not particularly limited, and examples thereof include excipients, disintegrants, lubricants, binders, coating agents, colorants, coagulation inhibitors, absorption promoters, solubilizing agents, stabilizers, health food materials, and nutritional supplement materials (supplement materials).

The excipients are not particularly limited, and examples of such excipients include white sugar, lactose, glucose, cornstarch, mannitol, crystalline cellulose, calcium phosphate, and calcium sulfate.

The disintegrants are not particularly limited, and examples of such disintegrants include starch, agar, calcium citrate, calcium carbonate, sodium hydrogen carbonate, dextrin, crystalline cellulose, carboxymethylcellulose, and tragacanth.

The lubricants are not particularly limited, and examples of such lubricants include talc, magnesium stearate, polyethylene glycol, silica, and hydrogenated vegetable oils.

The binders are not particularly limited, and examples of such binders include ethylcellulose, methylcellulose, hydroxypropymethyllcellulose, tragacanth, shellac, gelatin, gum arabic, polyvinylpyrrolidone, polyvinyl alcohol, polyacrylic acid, polymethacrylic acid, and sorbitol.

The coating agents are not particularly limited, and examples of such coating agents include gum arabic, Opadry, prunella spike, castor wax, carboxyvinyl polymers, carmellose, hydrous silicon dioxide, magnesium silicate, vinyl acetate resins, stearic acid, cetanol, and hydroxypropylmethylcellulose.

The colorants are not particularly limited, and pharmaceutically acceptable and/or food-acceptable ones may be used, for example.

The coagulation inhibitors are not particularly limited, and examples of such coagulation inhibitors include stearic acid, talc, light silicic anhydride, and hydrous silicon dioxide.

The absorption promoters are not particularly limited, and examples of such absorption promoters include higher alcohols, higher fatty acids, and surfactants such as glycerol fatty acid esters.

The solubilizing agents are not particularly limited, and examples of such solubilizing agents include organic acids such as fumaric acid, succinic acid, and malic acid.

The stabilizers are not particularly limited, and examples of such stabilizers include, for example, benzoic acid, sodium benzoate, and ethyl paraoxybenzoate.

The health food materials are not particularly limited, and examples of such health food materials include Kampo medicines (Chinese herbal medicines) (e.g. Irei-to, Unkei-to, Unsei-in, Ogi-kenchu-to, Oren-gedoku-to, Oren-to, Kakkon-to, Kami-kihi-to, Kami-shoyo-san, Kam-baku-taiso-to, Kikyo-to Kihi-to, Kumi-binro-to, Keigai-rengyo-to, Keishi-ka-shakuyaku-daio-to, Keishi-ka-shakuyaku-to, Keishi-ka-ryukotsu-borei-to, Keishi-to, Keishi-ninjin-to, Keishi-bukuryo-gan, Keihi-to, Koso-san, Goko-to, Goshaku-san, Gosha-jinki-gan, Gorin-san, Saikan-to, Saiko-ka-ryukotsu-borei-to, Saiko-keishi-kankyo-to, Saiko-keishi-to, Saiko-seikan-to, Saiboku-to, Sairei-to, Sansonin-to, Jiin-koka-to, Shigyaku-san, Shikunshi-to, Shimotsu-to, Sha-kanzo-to, Shakuyaku-kanzo-to, Juzen-taiho-to, Jumi-haidoku-to, Sho-kenchu-to, Sho-saiko-to, Sho-seiryu-to, Shofu-san, Shin'i-seihai-to, Shimpi-to, Shimbu-to, Seijo-bofu-to, Seisho-ekki-to, Seishin-renshi-in, Seihai-to, Sokei-kakketsu-to, Daio-kanzo-to, Daio-botampi-to, Dai-kenchu-to, Dai-saiko-to, Dai-saiko-to-kyo-daio, Dai-joki-to, Dai-bofu-to, Ji-daboku-ippo, Choi-joki-to, Choto-san, Choyo-to, Chorei-to, Chorei-to-go-shimotsu-to, Tsu-do-san, Tokaku-joki-to, Toki-inshi, Toki-kenchu-to, Toki-shakuyaku-san, Toki-to, Nichin-to, Nyoshin-san, Ninjin-to, Ninjin-yoei-to, Haino-san-kyu-to, Bakumondo-to, Hachimi-jio-gan, Hange-koboku-to, Hange-shashin-to, Byakko-ka-ninjin-to, Bukuryo-in, Bukuryo-in-go-hange-koboku-to, Heii-san, Boi-ogi-to, Bofu-tsusho-san, Hochu-ekki-to, Mao-to, Mao-bushi-saishin-to, Ma-kyo-kan-seki-to, Mashinin-gan, Moku-boi-to, Yoku-kan-san, Yoku-kan-san-ka-chimpi-hange, Rikkunshi-to, Rikko-san, Ryutan-shakan-to, Ryo-kan-kyo-mi-shin-ge-nin-to, and Rokumi-gan), tea leaves (e.g. green tea, Japanese tea mixed with roasted rice, powdered green tea, green tea of middle grade, toasted tea, roasted tea, jasmine tea, oolong tea, tea, black tea, flower tea, blue tea, and white tea), herbs (e.g. Italian parsley, elecampane, olive, oregano, cardoon, chamomile, curry plant, catnip, caraway, Christmas rose, crimson clover, cornflower, common mallow, salad burnet, santolina, cinnamon, jasmine, stevia, sage, linden (lime), scented geranium, St. John's wort, soapwort, Solomon's seal, thyme, tansy, chervil, chive, nasturtium, nutmeg, basil, honeysuckle, hyssop, flax, fennel, foxglove, black hollyhock, French marigold, betony, heliotrope, bergamot, hemp agrimony, rue, pot marigold, borage, white horehound, myrtle, mullein, marjoram, mint, yarrow, lavender, Lady's bedstraw, lemongrass, lemon verbena, lemon balm, rose, rosemary, rocket, wild strawberry, wild pansy, forget-me-not, etc.), pycnogenol, flavangenol, propolis, gingko leaves, royal jelly, carnitine, mushrooms, green juice, and extracts from these.

The nutritional supplement materials are not particularly limited, and examples of such nutritional supplement materials include amino acids, metal ions, proteins, saccharides, fatty acids, yeast extracts, vegetable extracts, fish meat extracts, fruits, and fruit extracts.

The composition of the invention may contain other antioxidants or vitamins.

The antioxidants are not particularly limited, and examples of such antioxidants include citric acid, citric acid derivatives, vitamin C, vitamin C derivatives, lycopene, vitamin A, carotenoids, vitamin B, vitamin B derivatives, flavonoids, polyphenols, glutathione, selenium, sodium thiosulfate, vitamin E, vitamin E derivatives, pyrroloquinoline quinone, pyrroloquinoline quinone derivatives, superoxide dismutase (SOD), glutathione peroxidase, glutathione-S-transferase, glutathione reductase, catalase, ascorbic acid peroxidase, and mixtures of these.

The vitamins are not particularly limited, and examples of such vitamins include vitamin A, vitamin B, vitamin B group, vitamin C, vitamin D, vitamin E, vitamin K, and derivatives thereof.

The dosage form of the composition for life extension of the invention may be either liquid or solid form. The method of administration may be any of various methods such as oral administration, injection, nasal administration, instillation administration, administration in the form of suppositories, or ingestion of coenzyme Q-containing foodstuffs. Generally, oral administration is considered to be most effective in view of dosage or the like. When oral administration is difficult, the composition for life extension of the invention can be administered by any other route than oral administration without any problems. For example, in patients or aged persons who find difficulty in orally taking nutrients, the recommendable methods of administration include, but are not limited to, administration in the form of suppositories, and external preparations for dermal application.

The composition for life extension of the invention extends the life span of target humans or animals by administering the composition to them. Examples of such animals include mammals, fishes, birds, reptiles and insects. Among them, mammals are preferred, and humans are particularly preferred. The term "life span" used herein means not only a period from birth to death, but also a period in which humans or animals can live in a vigorous and active condition. For example, ingestion of the composition for life extension of the present invention prevents or improves symptoms such as behavioral depressions accompanied with aging, including deteriorations of activity and passivity. Therefore, a human or an animal can live a healthy and active life even after he or it becomes older age. Although it is reported that ubiquinol suppresses deterioration in activity as one of effects of suppressing aging by ubiquinol (WO 2005/065672), this case shows an effect within an original life period. The present invention makes it clear for the first time that humans or animals can live a vigorous and active life in a further-extended life period.

In order to evaluate these life extension effects, a special senescence-accelerated model mouse (for example, SAMP8), which has a shorter lifetime than a common mouse, may be used as a model for evaluations in vivo, for example. This mouse is a model mouse which has been discovered and bread in Kyoto University, and remarkably shows aging states at an earlier stage. This mouse is very useful model for evaluating effects over a life span. The aging states shown in this mouse extremely resembles the aging states of humans, and is also useful to evaluate the health condition of humans in a senectitude.

In the case of orally administrating the composition of the present invention, for example, the dose of the composition of the invention as expressed in terms of the amount of reduced coenzyme Q is preferably 30 to 1,200 mg, more preferably 50 to 800 mg, still more preferably 100 to 300 mg, per day per human. For an animal other than a human, the composition may be administered in an amount calculated by converting the above preferred dose into an amount per weight.

The composition for life extension of the present invention preferably contains reduced coenzyme Q at a proportion of 0.001 to 99% by weight, more preferably 0.01 to 70% by weight, and still more preferably 0.1 to 50% by weight to the whole composition.

### EXAMPLES

The present invention will be explained in more detail by illustrating Examples. These Examples are, however, by no means limitative of the scope of the invention. The purity of coenzyme Q₁₀, and the ratio (weight ratio) of [(reduced coenzyme Q₁₀) / (reduced coenzyme Q₁₀ + oxidized coenzyme Q₁₀)] were determined by the following HPLC analysis.

### (HPLC analysis condition)

Column: SYMMETRY C18 (a product of Waters), 250 mm (length), 4.6 mm (inside diameter)
Mobile phase: C₂H₅OH:CH₃OH = 4:3 (v:v)
Detection wavelength: 210 nm
Flow rate: 1 ml/min
Retention time of reduced coenzyme Q₁₀: 9.1 min, Retention time of oxidized coenzyme Q₁₀: 13.3 min

### (Production Example 1) Production of reduced coenzyme Q₁₀

Into 1000 g of ethanol, 100 g of oxidized coenzyme Q₁₀ (purity: 99.4%, a product of Kaneka Corporation) and 60 g of L-ascorbic acid were added, and the mixture was stirred at 78°C, to allow reduction reaction to be proceeded. After a lapse of 30 hours from the start of stirring, the mixture was cooled to 50°C, and 330 g of ethanol and 70 g of water were added to the mixture with holding the temperature. With stirring this ethanol solution (which contained 100 g of reduced coenzyme Q₁₀), the solution was cooled to 2°C at a cooling rate of 10°C /hour, to give white slurry. The resultant slurry was filtered under reduced pressure, and the recovered wet crystal was washed with cold ethanol, cold water, and cold ethanol in this order (the temperature of the cold solvents used for washing was 2°C). The washed wet crystal was dried under reduced pressure (20 to 40°C, 1 to 30 mmHg), to give 97 g of a white dried crystal (purity: 99%, the ratio of [(reduced coenzyme Q₁₀) / (reduced coenzyme Q₁₀ + oxidized coenzyme Q₁₀)] = about 99% by weight). All the operations except drying under reduced pressure were carried out under a nitrogen atmosphere.

### (Production Example 2) Production of reduced coenzyme Q₁₀

In 1,000 g of heptane solution, 100 g of oxidized coenzyme Q₁₀ (purity: 99.4%, a product of Kaneka Corporation) was dissolved at 25°C. While stirring, an aqueous solution prepared by dissolving 100 g of sodium dithionite (purity: not lower than 75%), as a reducing agent, in 1,000 ml of water was gradually added in the heptane solution, thus allowing the reduction reaction to proceed at pH 4 to 6 at 25°C. After the lapse of 2 hours, the aqueous phase was removed from the reaction mixture, and the heptane phase was washed with 1,000 g of a deaerated saturated aqueous solution of sodium chloride for six times. All the above operations were carried out under a nitrogen atmosphere. This heptane phase was subjected to solvent substitution under reduced pressure, to prepare a 7% (w/w) ethanol solution of reduced coenzyme Q₁₀ at 50°C (the solution containing 100 g of reduced coenzyme Q₁₀). In this ethanol solution, 50 g of water was added, and the mixture was cooled to 2°C at a cooling rate of 10°C/hour while stirring, to precipitate crystal. All the operations were carried out in a nitrogen atmosphere. The slurry thus obtained was filtered under reduced pressure, and the recovered wet crystals were washed with cold ethanol, cold water and cold ethanol in this order (the temperature of the cold solvents used for washing was 2°C). The washed wet crystal was dried under reduced pressure (20 to 40°C, 1 to 30 mmHg), to give 97 g of a white dried crystal (purity: 99%, the ratio of [(reduced coenzyme Q₁₀) / (reduced coenzyme Q₁₀ + oxidized coenzyme Q₁₀)] = about 99% by weight).

### (Example 1) The life extending effect for a senescence-accelerated model mouse

Three groups were set as follows. One is a group, to which feedstuff (CE-2, a product of CLEA Japan, Inc.) containing 0.4% of reduced coenzyme Q₁₀ produced in Production Example 1 (containing about 1% by weight of oxidized coenzyme Q₁₀) was given. Another group is a group, to which feedstuff (CE-2, a product of CLEA Japan, Inc.) containing 0.4% of commercially-available oxidized coenzyme Q₁₀ (100% oxidized coenzyme Q₁₀, a product of Kaneka Corporation) was given. The rest is a control group, to which only basic feedstuff (CE-2, a product of CLEA Japan, Inc.) was given. Each group consisted of 8 to 10 senescence-acceleratedmodel mice (SAMP8, 3-week old, male). Each group was allowed free access to the feedstuff. During the test period, measurements of motor activities and recording of the number of dead mice were periodically performed. The dose of reduced and oxidized coenzyme Q₁₀ as estimated from the feed consumption and animal body weight corresponded to about 300 to 500 mg/kg/day.

Results are illustrated in Tables 1 and 2. Table 1 illustrates the survival rates in the control group, the reduced coenzyme Q₁₀-ingested group, and the oxidized coenzyme Q₁₀-ingested group at the 13th month after the start of the test. Each data consists of n= 8 to 10. The survival rate was assayed by the Kaplan-Meier method. The symbol "*" expresses that there is a significant difference relative to the control group at the degree of risk of 5%. Table 2 illustrates averages of motor activities (measuring apparatus: SUPERMEX, a product of Muromachi machinery) over a period from 16 o'clock to 9 o'clock in the morning of next day, and the relative proportions to the control group, estimated by assuming the level of the control group as 100. The data illustrated in Table 2 are values for each of the control group, the reduced coenzyme Q₁₀-ingested group, and the oxidized coenzyme Q₁₀-ingested group at the 11th month after the start of the test. Each data consist of n= 4 to 7, and significant difference was tested by t-test. The p values are listed in the Table 2.

**Table 1**

| | Control group | Reduced coenzyme Q₁₀-ingested group | Oxidized coenzyme Q₁₀- ingested group |
|---|---|---|---|
| Survival Rate (%) | 11 | 75 | 40 |
| Significant difference test | | * | * |

**Table 2**

| | Control group | Reduced coenzyme Q₁₀-ingested group | Oxidized coenzyme Q₁₀-ingested group |
|---|---|---|---|
| Motor activities | 10753 | 17520 | 12754 |
| (Count) | (100) | (163) | (119) |
| Significant difference test | | p= 0.058 | p= 0.75 |

From the above results, it was found that oxidized coenzyme Q₁₀ slightly improved extension of a life span and inhibition of deterioration of the motor activity. However, remarkable extension of a life span and inhibition of deterioration of the motor activity were exerted as a result of ingestion of reduced coenzyme Q₁₀. Such effects cannot be expected from the effect of oxidized coenzyme Q₁₀.

### (Preparation Example 1) Powder

Reduced coenzyme Q₁₀ (containing about 1% of oxidized coenzyme Q₁₀) was dissolved in propanol and allowed to be adsorbed on microcrystalline cellulose, which was then dried under reduced pressure. This was mixed with cornstarch in a nitrogen flow to give a powder preparation.

| | |
|---|---|
| Reduced coenzyme Q₁₀ | 9.9 parts by weight |
| Oxidized coenzyme Q₁₀ | 0.1 part by weight |
| Microcrystalline cellulose | 40 parts by weight |
| Cornstarch | 55 parts by weight |

### (Preparation Example 2) Capsules

Using the materials specified below, a powder preparation was prepared in the same manner as in Preparation Example 1. This powder was filled into gelatin capsules in the conventional manner. The filled capsules were sealed and packed in a nitrogen atmosphere and stored in a refrigerator.

| | |
|---|---|
| Reduced coenzyme Q₁₀ | 19.8 parts by weight |
| Oxidized coenzyme Q₁₀ | 0.2 part by weight |
| Microcrystalline cellulose | 40 parts by weight |
| Cornstarch | 20 parts by weight |
| Lactose | 65 parts by weight |
| Magnesium stearate | 3 parts by weight |
| Polyvinylpyrrolidone | 2 parts by weight |

### (Preparation Example 3) Soft capsules

Corn oil was warmed to 50°C. Thereto was added with reduced coenzyme Q₁₀ (containing about 1% of oxidized coenzyme Q₁₀) melted at the same temperature for dissolution. The solution was encapsulated into soft capsules in the conventional manner.

| | |
|---|---|
| Reduced coenzyme Q₁₀ | 49.5 parts by weight |
| Oxidized coenzyme Q₁₀ | 0.5 part by weight |
| Corn oil | 350 parts by weight |

### (Preparation Example 4) Tablets

Reduced coenzyme Q₁₀ (containing about 1% of oxidized coenzyme Q₁₀) was dissolved in propanol and allowed to be adsorbed on microcrystalline cellulose, which was then dried under reduced pressure. This was mixed with cornstarch, lactose, carboxymethylcellulose calcium and magnesium stearate in a nitrogen atmosphere, an aqueous solution of polyvinylpyrrolidone was then added as a binder, to the resulting mixture, and the whole mixture was granulated in the conventional manner. To this granulation product, talc was added and mixed as a lubricant, and then the resulting mixture was made into tablets. The tablets were packed in a nitrogen atmosphere and stored in a refrigerator.

| | |
|---|---|
| Reduced coenzyme Q₁₀ | 19.8 parts by weight |
| Oxidized coenzyme Q₁₀ | 0.2 part by weight |
| Cornstarch | 25 parts by weight |
| Lactose | 15 parts by weight |
| Carboxymethylcellulose calcium | 10 parts by weight |
| Microcrystalline cellulose | 40 parts by weight |
| Polyvinylpyrrolidone | 5 parts by weight |
| Magnesium stearate | 3 parts by weight |
| Talc | 10 parts by weight |

## Claims

1. A composition for life extension,
which comprises reduced coenzyme Q represented by the following general formula (1): 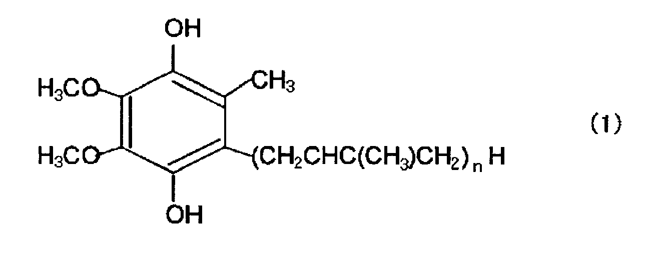 wherein n is an integer of 1 to 12;
as an active ingredient.

2. The composition according to claim 1,
wherein the reduced coenzyme Q is reduced coenzyme Qᵢₒ.

3. The composition according to claim 1,
wherein the amount of the reduced coenzyme Q in the composition is 0.001 to 99% by weight.

4. The composition according to claim 1,
which further comprises oxidized coenzyme Q represented by the following general formula (2): 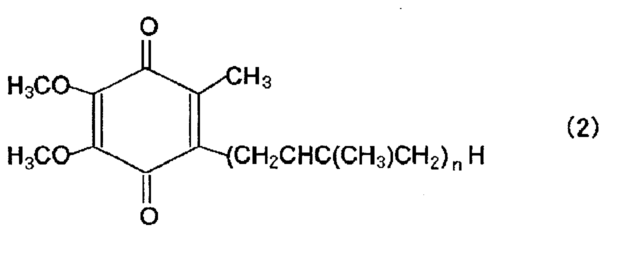 wherein n is an integer of 1 to 12.

5. The composition according to claim 4,
wherein the oxidized coenzyme Q is oxidized coenzyme Q₁₀.

6. A method for extending a life span,
which comprises administering a composition comprising reduced coenzyme Q represented by the following general formula (1) : 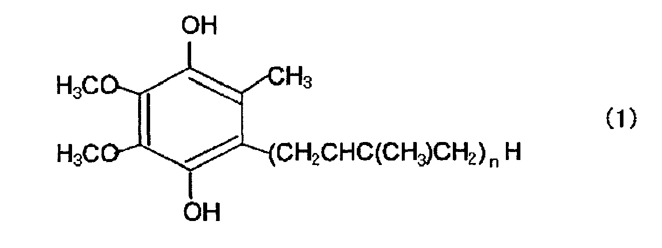 wherein n is an integer of 1 to 12;
as an active ingredient.

7. The method according to claim 6,
wherein the reduced coenzyme Q is reduced coenzyme Q₁₀.

8. The method according to claim 6,
wherein the amount of the reduced coenzyme Q in the composition is 0.001 to 99% by weight.

9. The method according to Claim 6,
wherein the composition further comprises oxidized coenzyme Q represented by the following general formula (2) : 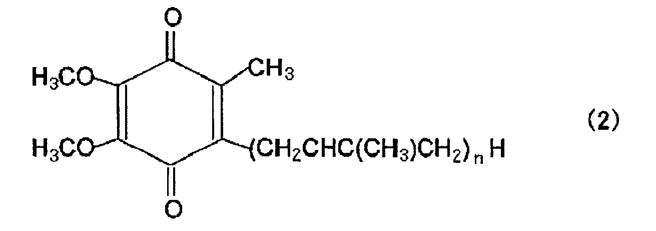 wherein n is an integer of 1 to 12.

10. The method according to claim 9,
wherein the oxidized coenzyme Q is oxidized coenzyme Q₁₀.

11. The method according to any one of claims 6 to 10,
wherein a subject to be administered is a mammal.
